# EUROPEAN PATENT SPECIFICATION

(11) **EP 0 713 377 B1**
(45) Date of publication and mention of the grant of the patent: **28.04.1999**
(21) Application number: 93921530.7
(22) Date of filing: 17.09.1993
(51) Int. Cl.: A61F 9/00, A61F 2/14

(54) **METHOD FOR PREPARATION OF VOLUTE GRAFTS AND GRAFT**
VERFAHREN ZUR BEREITUNG VON GEWICKELTEN TRANSPLANTATEN UND TRANSPLANTAT
PROCEDE DE PREPARATION ET DE GREFFONS EN VOLUTE ET GREFFON EN VOLUTE

(30) Priority: 16.03.1993 US 33105; 30.04.1993 US 57144
(43) Date of publication of application: 29.05.1996
(73) Proprietor: PHOTOGENESIS Incorporated, Los Angeles, CA 90069 (US)
(72) Inventor: SILVERMAN, Martin, S., St. Louis, MO 63110 (US); HUGHES, Stephen E., Delmar, New York 12054 (US)
(74) Representative: Littolff, Denis
(86) International application number: US9308616
(87) International publication number: WO9421205

(56) References cited:
- EP-A- 0 340 698
- EP-A- 0 535 506
- WO-A-91/02499
- WO-A-92/08406
- DE-A- 4 004 921

## Description

### BACKGROUND OF THE INVENTION

The present invention relates in general to surgical techniques.

The retina is the sensory epithelial surface that lines the posterior aspect of the eye, receives the image formed by the lens, transduces this image into neural impulses and conveys this information to the brain by the optic nerve. The retina comprises a number of layers, namely, the ganglion cell layer, inner plexiform layer, inner nuclear layer, outer plexiform layer, outer nuclear layer, photoreceptor inner segments and outer segments. The outer nuclear layer comprises the cell bodies of the photoreceptor cells with the inner and outer segments being extensions of the cell bodies.

The choroid is a vascular membrane containing large branched pigment cells that lies between the retina and the sclerotic coat of the vertebrate eye. Immediately between the choroid and the retina is the retinal pigment epithelium which forms an intimate structural and functional relationship with the photoreceptor cells.

Several forms of blindness are primarily related to the loss of photoreceptor cells caused by defects in the retina, retinal pigment epithelium, choroid or possibly other factors (e.g. intense light, retinal detachment, intraocular bleeding). In several retinal degenerative diseases select populations of cells are lost. Specifically, in macular degeneration and retinitis pigmentosa, the retinal photoreceptors degenerate while other cells in the retina as well as the retina's central connections are maintained. In an effort to recover what was previously thought to be an irreparably injured retina, researchers have suggested various forms of grafts and transplantation techniques, none of which constitute an effective manner for reconstructing a dystrophic retina.

The transplantation of retinal cells to the eye can be traced to a report by Royo et al., Growth 23: 313-336 (1959) in which embryonic retina was transplanted to the anterior chamber of the maternal eye. A variety of cells were reported to survive, including photoreceptors. Subsequently del Cerro was able to repeat and extend these experiments (del Cerro et al., Invest. Ophthalmol. Vis. Sci. 26: 1182-1185, 1985). Soon afterward Turner, et al. Dev. Brain Res. 26:91-104 (1986) showed that neonatal retinal tissue could be transplanted into retinal wounds.

In related studies, Simmons et al., Soc. Neurosci. Abstr. 10: 668 (1984) demonstrated that embryonic retina could be transplanted intracranially, survive, show considerable normal development, be able to innervate central structures, and activate these structures in a light-dependent fashion. Furthermore, these intracranial transplants could elicit light-dependent behavioral responses (pupillary reflex) that were mediated through the host's nervous system. Klassen et al., Exp. Neurol. 102: 102-108 (1988) and Klassen et al. Proc. Natl. Acad., Sci. USA 86:6958-6960 (1987).

Li and Turner, Exp. Eye Res. 47:911 (1988) have proposed the transplantation of retinal pigment epithelium (RPE) into the subretinal space as a therapeutic approach in the RCS dystrophic rat to replace defective mutant RPE cells with their healthy wild-type counterparts. According to their approach, RPE was isolated from six- to eight-day old black eyed rats and grafted into the subretinal space by using a lesion paradigm which penetrates through the sclera and choroid. A 1 ml injection of RPE (40,000 - 60,000 cells) was made at the incision site into the subretinal space by means of a 10 ml syringe to which was attached a 30 gauge needle. However, this method destroys the cellular polarity and native organization of the donor retinal pigment epithelium which is desirable for transplants.

del Cerro, (del Cerro et al., Invest. Ophthalmol. Vis. Sci. 26: 1182-1185, 1985) reported a method for the transplantation of tissue strips into the anterior chamber or into the host retina. The strips were prepared by excising the neural retina from the donor eye. The retina was then cut into suitable tissue strips which were then injected into the appropriate location by means of a 30 gauge needle or micropipette with the width of the strip limited to the inner diameter of the needle (250 micrometers) and the length of the strip being less than 1 millimeter. While del Cerro reports that the intraocular transplantation of retinal strips can survive, he notes that the procedure has some definite limitations. For instance, his techniques do not allow for the replacement of just the missing cells (e.g. photoreceptors) but always include a mixture of retinal cells. Thus, with such a transplant appropriate reconstruction of the dystrophic retina that lacks a specific population of cells (e.g., photoreceptors) is not possible.

del Cerro et al., Neurosci. Lett. 92: 21-26, 1988, also reported a procedure for the transplantation of dissociated neuroretinal cells. In this procedure, the donor retina is cut into small pieces, incubated in trypsin for 15 minutes, and triturated into a single cell suspension by aspirating it through a fine pulled pipette. Comparable to the Li and Turner approach discussed above, this procedure destroys the organized native structure of the transplant, including the donor outer nuclear layer ; the strict organization of the photoreceptors with the outer segments directed toward the pigment epithelium and the synaptic terminals facing the outer plexiform layer are lost. Furthermore, no means of isolating and purifying any given population of retinal cells (e.g. photoreceptors) from other retinal cells was demonstrated.

It is believed by the present inventor that it is necessary to maintain the photoreceptors in an organized outer nuclear layer structure in order to restore a reasonable degree of vision. This conclusion is based on the well known optical characteristics of photoreceptors (outer segments act as light guides) and clinical evidence showing that folds or similar, even minor disruptions in the retinal geometry can severely degrade visual acuity.

Document WO-A-91/02499 discloses a graft for transplantation into the subretinal area of a host eye, said graft comprising a carrier layer of a non toxic flexible composition which substantially dissolves in the host eye and a donor layer harvested from a donor eye, said donor layer comprising eye tissue selected from retinal tissue, epithelial tissue, choroidal tissue, and Bruch's membrane or a synthetic equivalent, said donor layer having the same cellular organization as in the donor eye. The same document also discloses a method for preparing an implant having such a composition and a sheet like configuration.

This invention comprises a new and improved configuration for an implant of this type and a new and improved method for preparing such new implant.

### SUMMARY OF THE INVENTION

Among the objects of the present invention, therefore, may be noted the provision of a method which conserves relatively large expanses of tissue harvested from a donor eye ; the provision of such a method in which a relatively large expanse of harvested tissue is so formed as to enable the harvested tissue to be inserted into a standard-sized incision in the eye ; the provision of such a method in which the polarity and organization of the cells at the time of harvest are maintained in the graft ; and the provision of a method for implantation of grafts to the subretinal area of an eye.

Further among the several objects and features of the present invention may be noted the provision of a graft for use in the reconstruction of a dystrophic retina or rescue of endogenous photoreceptor cells of an individual afflicted with an inherited or acquired retinal disease which causes a progressive loss of rods and subsequent eventual cone dystrophy, dysfunction and/or loss ; the provision of such a graft which facilitates regrowth of photoreceptor axons by maintaining the polar organization of the photoreceptor and the close proximity of their postsynaptic targets with the adjacent outer plexiform layer upon transplantation.

These objects are met by the method of preparing an implant according to claim 1 and by the graft according to claim 2.

Generally, the implantation method comprises coiling an implantable material which is of sheet-like form to form a volute. The convolutions of the volute are free of one another for subsequent uncoiling of the implantable material substantially to its original sheet-like form. An incision is made in the host eye for the insertion of the volute to a position between the retina and the underlying tissue of the host eye. The incision is smaller than the incision that would be required for insertion of the implantable material in its uncoiled sheet-like form. The volute is inserted one end first into the host eye through the incision to a position between the retina and the underlying tissue. The volute uncoils after its insertion to lie in sheet-like form between the retina and the underlying tissue of the host eye and the incision is closed.

Generally, the graft for implantation comprises a layer of a non-toxic flexible composition which substantially dissolves at body temperature and a material to be implanted coiled to form a volute. The volute is insertable one end first through the incision dimensioned in accordance with the cross-sectional area of the volute to a position for implantation, and then uncoiled to lie in sheet-like form at the site of implantation.

Other objects and features of the invention will be in part apparent and in part pointed out hereinafter.

### BRIEF DESCRIPTION OF THE DRAWINGS

Fig. 1 is a photograph of a cryostat section of normal rat retina as set forth in Example 1 ;
Fig. 2 is a photograph of a blinded rat retina following constant illumination as set forth in Example 1 ;
Fig. 3 is a schematic of a donor retina ;
Fig. 4 is a schematic of a flattened retina ;
Fig. 5 is a schematic of a flattened retina mounted to a substrate ;
Fig. 6 is a schematic of a sectioned retina mounted to a substrate ;
Fig. 7 is a schematic of a laminate comprising a retina section on a supporting, stabilizing substrate ;
Fig. 8 is a schematic top plan view of the laminate of Fig. 7, showing a graft (dashed lines) comprising a photoreceptor cell layer and a supporting, stabilizing substrate ;
Fig. 9 is a schematic of the graft mounted on a plate formed with spacers ;
Fig. 10 is a schematic of the graft mounted on a plate infused with molten gelatin with a cover plate ;
Fig. 11 is a schematic of the top plate being laterally slid off ;
Fig. 12 is a schematic of the resulting graft ;
Fig. 13 is a schematic of the graft being skived ;
Fig. 14 is a schematic of the skived graft being removed from the plate for transplantation ;
Fig. 15 is a perspective view of a volute.

### DETAILED DESCRIPTION

As used herein, the term "donor" shall mean the same or different organism relative to the host and the term "donor tissue" shall mean tissue harvested from the same or different organism relative to the host.

Several forms of blindness such as retinitis pigmentosa, retinal detachment, macular degeneration, and light exposure-related blindness, are primarily related to the loss of the photoreceptors in the eye. However, destruction of the photoreceptors does not necessarily lead to the loss of the remaining retina or axons that connect the retina to the brain. Surprisingly, it has been discovered that some degree of vision can be restored by replacing damaged photoreceptors with photoreceptors harvested from a donor and which are maintained in their original organization and cellular polarity.

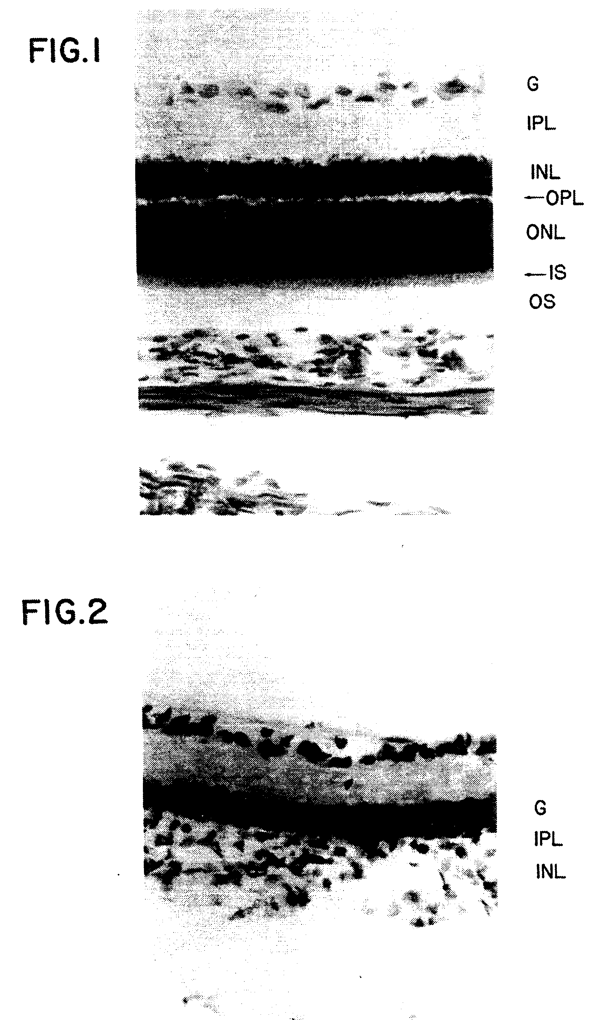

Fig. 1 is a photograph of a cryostat section of normal rat retina. Fig. 2 is a photograph of a cryostat section of a rat retina following constant illumination which destroys the photoreceptor (outer nuclear) layer while leaving other retinal layers and cells largely intact. In these and subsequent figures, the retina or layers thereof, e.g., the ganglion cell layer ("G"), inner plexiform layer ("IPL"), inner nuclear layer ("INL"), outer plexiform layer ("OPL"), outer nuclear layer ("ONL"), inner segments ("IS"), outer segments ("OS"), and retinal pigment epithelium ("RPE"), are shown, respectively, from top to bottom.

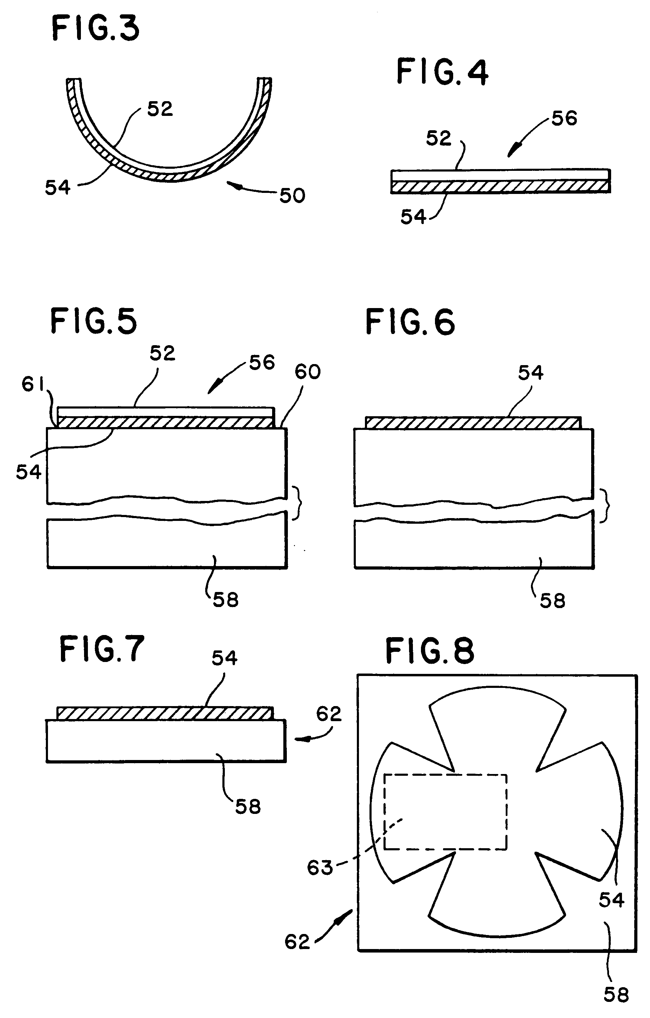

Referring now to Fig. 3, a photoreceptor graft for implantation through an incision smaller than the width of the graft in sheet-like form is prepared in accordance with a method of the present invention. The graft, however, may comprise other implantable material such as other retinal cells, antiviral and antibiotic agents and/or other pharmacologic agents.

A graft comprising photoreceptor cells is prepared by removing a donor retina 50 comprising inner retina layers 52 and a photoreceptor layer 54 from a donor eye. The donor retina 50 is flattened (Fig. 4) by making a plurality of cuts through the retina from locations near the center of the retina to the outer edges thereof (see Fig. 8). Cuts can be made in other directions if necessary.

As shown in Fig. 5, the flattened retina 56 is placed with the photoreceptor side 54 down on a gelatin slab 58 which has been surfaced so as to provide a flat surface 60 that is parallel to the blade of a vibratome apparatus. The gelatin slab 58 is secured to a conventional vibratome chuck of the vibratome apparatus. Molten four to five per cent gelatin solution is deposited adjacent the flattened retina/gelatin surface interface 61 and is drawn by capillary action under the flattened retina 56 causing the flattened retina to float upon the gelatin slab 58. Excess molten gelatin is promptly removed and the floating flattened retina 56 is then cooled to approximately 4°C with ice-cold Ringer's solution that surrounds the gelatin block to cause the molten gelatin to gel. The flattened retina 56 is thereby adhered to the gelatin block 58.

As shown in Fig. 6, the inner retina portion 52 is sectioned from the top down at approximately 20 to 50 millimicrons until the photoreceptor layer 54 is reached, thereby isolating the photoreceptor layer from the inner layers of the retina, i.e., the ganglion cell layer, inner plexiform layer, inner nuclear layer, and outer plexiform layer. When the photoreceptor layer 54 is reached, the vibratome stage is advanced and a section from approximately 50 to 300 millimicrons thick is obtained as shown in Fig. 7. The thickness of this section should be sufficient to undercut the photoreceptor and form a section 62 consisting of a layer of photoreceptor cells and a thin gelatin substrate 58 adhered thereto. As shown in Fig. 8, the section 62 is cut vertically along the dashed lines to create a laminate 63.

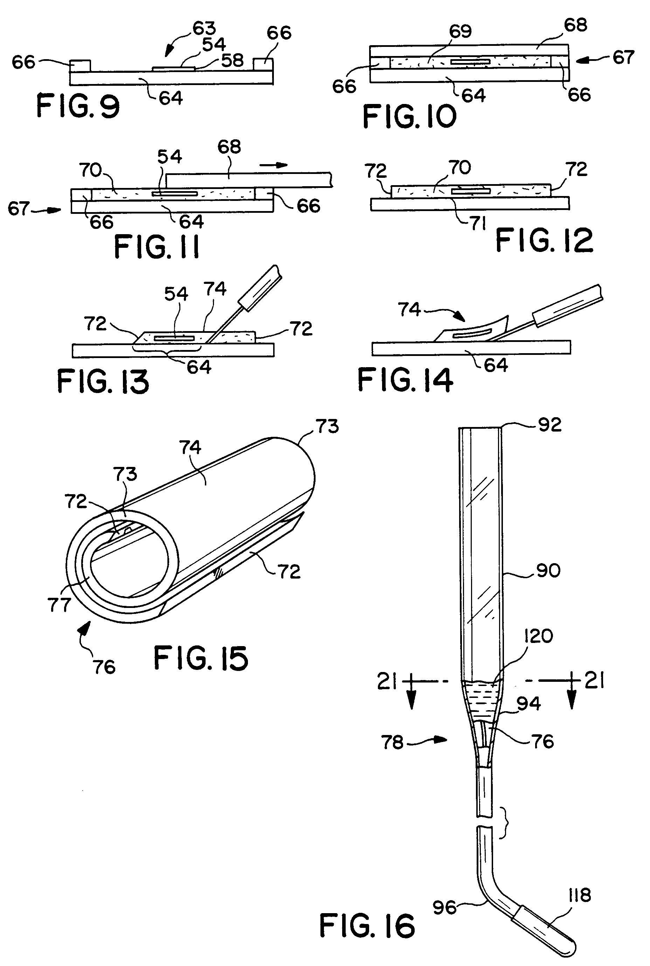

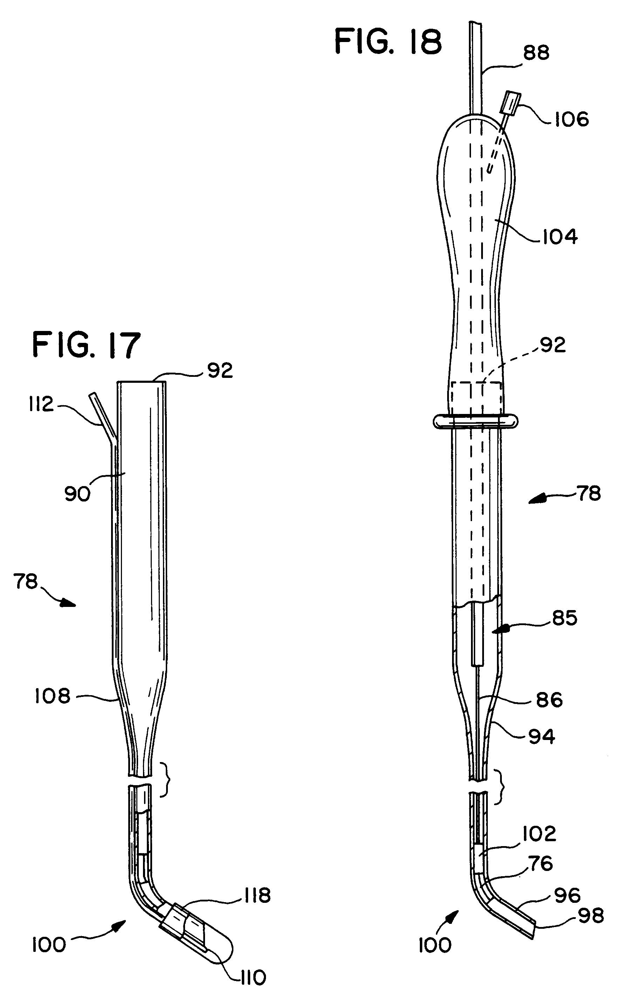

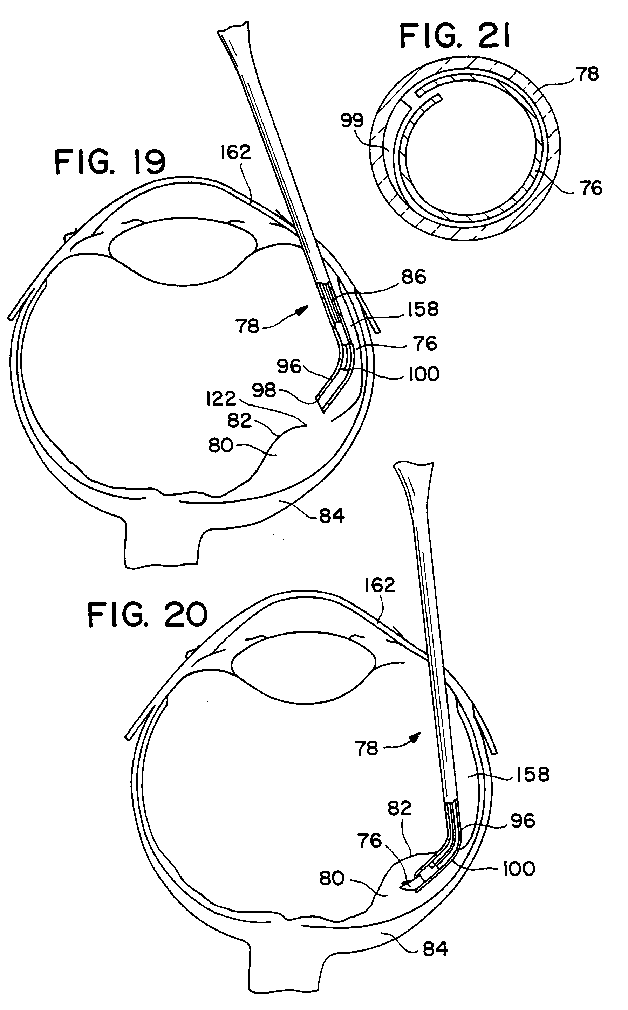

The laminate 63 is then placed onto a flat plate 64 formed with risers 66 as shown in Fig. 9. The plate 64, with the laminate 63 positioned between the risers 66, is infused with molten fifteen to twenty per cent gelatin solution to surround and cover the photoreceptor layer 54 with the gelatin substrate 58 is surrounded and covered by the molten gelatin. As shown in Fig. 10, a flat cover plate 68 is placed on top of the risers 66 to remove any excess molten gelatin and to establish the precise thickness of the graft. The height of the risers 66 can be adjusted to prepare grafts of different thicknesses.

The resulting container 67 consisting of two plates 64, 68 separated by risers 66 encasing a gelatin slab 69 with the photoreceptor layer 54 embedded therein is cooled to room temperature to cause the molten gelatin to gel and form a carrier sheet 70 encapsulating the photoreceptor layer 54. The outer segment (not shown) of the photoreceptor layer 54 faces toward one face 71 of the carrier sheet 70.

As shown in Fig. 11, after the molten gelatin is allowed to gel, the top cover plate 68 of the laminate is carefully removed by sliding the plate laterally away from the risers 66 so as to prevent any tearing of the gelatin carrier sheet 70 and layer of photoreceptors 54. The risers 66 are likewise removed to expose the carrier sheet 70. To further reduce the risk of tearing the gelatin carrier sheet 70 upon removal of the top cover plate 68 the top cover plate can be wrapped in a TEFLON (registered Trade Mark) film (not shown) so that the bottom surface of the cover plate has a smooth layer of film affixed thereto. The top cover plate is removed by unwrapping the film on the upper surface of the cover plate and lifting the plate from the risers 66. The TEFLON (registered Trade Mark) film is then carefully peeled from the gelatin carrier sheet 70. Immersion in a dissecting fluid (such as an aqueous solution) can facilitate peeling.

Opposite ends 72 of the carrier sheet 70 are cut vertically to a size appropriate for transplantation. As shown in Fig. 13, opposite sides 72 of the carrier sheet 70 can be skived--cut at obtuse and acute angles relative to the top and bottom surfaces of the gelatin slab--to produce a graft 74 having approximately parallel sides. The skived sides 72 of the graft 74 facilitate the sliding of one side 72 of the graft over the other side. The surface of the graft 74 should have a surface area greater than about 1 square millimeter, preferably greater than 4 square millimeters or as large as may be practically handled within a surgical instrument for implantation of the graft through an incision in a host eye. Thus constructed, the graft 74 may subtend a considerable extent of the retinal surface.

To prepare the graft for insertion into the eye, the graft 74 is removed from the plate 64 (Fig. 14) and formed into a volute 76 (Fig. 15) having overlapping sides 72 and convolutions 77. The convolutions 77 of the volute 76 are free of one another in the sense that the convolutions do not impede the volute from subsequent uncoiling. Although it is not presently preferred, the sides 72 of the volute 76 do not necessarily need to overlap ; any coiled configuration of the graft 74 whereby the diameter of the volute is less than the distance between the sides 72 of the uncoiled, sheet-like graft and whereby the photoreceptor layer 54 is not damaged may be prepared in accordance with the present invention.

The thickness of the graft 74 comprising the sectioned flattened retinal tissue 54 and the carrier sheet 70 as discussed above is only approximate and will vary as donor material varies. In addition, sectioning may be facilitated and vibratome thickness further calibrated from histological measurements of the thickness of the retina, thereby providing further guides to sectioning depth. Appropriate sectioning thicknesses or depth may be further determined by microscopic examination and observation of the sections.

The gelatin carrier sheet 70 adds mechanical strength and stability to the easily damaged photoreceptor layer 54. As a result, the flattened retinal tissue 54 is less likely to be damaged and is more easily manipulated during the transplantation procedure. Gelatin is presently preferred as an encapsulant because of its flexibility, pliability, ability to dissolve at body temperature and apparent lack of toxicity to neural tissue upon dissolution.

However, other compositions such as auger or agarose which also have the desirable characteristics of gelatin may be substituted. Significantly, gelatin has not been found to interfere with tissue growth or post-transplant interaction between the graft 74 and the underlying retinal pigment epithelium. Gelatin is also presently preferred as an adhesive to laminate the retinal tissue 54 within the encapsulant. However, other compositions, including lectins such as concanavalin A, wheat germ agglutin, or photo reactive reagents which gel or decompose upon exposure to light and which also have the desirable characteristics of gelatin may be substituted as the adhesive.

Advantageously, the gelatin carrier sheet 70 or other encapsulant may additionally serve as a carrier for any of a number of trophic factors such as fibroblast growth factor, pharmacologic agents including immunosuppressants such as cyclosporin A, anti-inflammation agents such as dexamethasone, anti-angiogenic factors, anti-glial agents, and anti-mitotic factors. Upon dissolution of the encapsulant, the factor or agent becomes available to impart the desired effect upon the surrounding tissue. The dosage can be determined by established experimental techniques. The encapsulant may contain biodegradable polymers to act as slow release agents for pharmacologic substances that may be included in the encapsulant.

As an alternative to mechanical, e.g., microtome, sectioning, the donor retina 50 may be chemically sectioned. Specifically, it is known that neurotoxic agents such as kainic acid or anoxia are toxic to cells in ail retinal layers 52 except to photoreceptors and Müller cells. Therefore if the donor retina 50 is treated with an appropriate neurotoxic agent the photoreceptor layer 54 can be isolated. This technique has the advantage of maintaining the retinal Müller cells (which are relatively insensitive to kainic acid and anoxia) with the photoreceptor cells 54. Since it is known that Müller cells help maintain photoreceptor cells 54 (both biochemically and structurally) the isolation of Müller cells along with the photoreceptor cells could be advantageous.

If desired, the graft 74 may contain retinal pigment epithelial cells. Because the RPE is tenuously adherent to the retina, mechanical detachment of the retina from a donor eye ordinarily will cause the RPE to separate from the retina and remain attached to the choroid. However, through the use of enzymatic techniques such as those described in Mayerson et al., Invest. Opthalmol. Vis. Sci. 25: 1599-1609, 1985, the retina can be separated from the donor eye with the RPE attached. Alternatively, implants comprising a monolayer of RPE cells can be prepared by harvesting RPE cells from donor tissue and apposing the harvested RPE cells as an intact monolayer to a non-toxic, flexible composition, or by seeding such a composition with a monolayer of dissociated RPE cells and allowing them to grow into a confluent layer. The flexible composition serves as a stabilizing support for the RPE cells during encapsulation and transplantation.

Grafts comprising the choroid, Bruch's membrane or a synthetic Bruch's membrane (e.g., collagen sheet on the order of 1-5 microns) may also be prepared. The choroid is stripped off of the scleral lining of the eye (with or without the RPE attached) and flattened by making radial cuts. The donor choroid may be encapsulated as previously described for the photoreceptor cells and/or combined with a photoreceptor layer 54 which has been prepared as described above to form a laminate comprising a photoreceptor layer and a choroidal layer encapsulated within a gelatin substrate and superstrate.

As shown and described in European Parent Application EP-A-0 486 589, a trans-choroidal, scleral and corneal surgical approach may be used as an alternative to the pars plana approach described above. Except for the point of entry, the surgical technique is essentially the same as outlined above. In view of the above, it will be seen that the several objects of the invention are achieved and other advantages attained.

As various changes could be made in the above surgical instruments, compositions of matter and methods without departing from the scope of the invention as claimed, it is intended that all matter contained in the above description or shown in the accompanying drawings shall be interpreted as illustrative and not in a limiting sense.

## Claims

1. A method of preparing an implant (74) having a sheet-like configuration for implantation into the subretinal area of a host eye, wherein said implant comprises a carrier layer (70) of a non-toxic flexible composition which substantially dissolves in the host eye, and a donor layer (54) harvested from a donor eye, said donor layer (54) comprising eye tissue selected from retinal tissue, epithelial tissue, choroidal tissue, and Bruch's membrane or a synthetic equivalent, said donor layer (54) having the same cellular organization as in the donor eye,
characterized by the steps of :
- providing a tubular body (90) having a first open end (92) and a second open end (96), the diameter of said first open end being larger than the diameter of said second open end, wherein the bore of said tubular body includes a funnel shaped portion (94) leading from said first open end towards said second open end ;
- providing said implant (74) having a sheet-like configuration ; and
- coiling said sheet-like implant (74) to form a volute implant (76) by placing the sheet-like implant one end first in the first open end (92) of said tubular body, feeding the sheet-like implant through said tubular body (90) towards said second open end (96), whereby engagement of said sheet-like implant (74) with the surface of said bore as it passes through said funnel shaped portion (94) coils the sheet-like implant into a volute implant (76), wherein said volute implant (76) may exit from said second end (96).

2. A graft for transplantation into the subretinal area of a host eye, wherein said graft comprises a carrier layer (70) of a non-toxic flexible composition which substantially dissolves in the host eye, and a donor layer (54) harvested from a donor eye, said donor layer (54) having the same cellular organization as in the donor eye, said donor layer (54) comprising eye tissue selected from retinal tissue, epithelial tissue, choroidal tissue, and Bruch's membrane or a synthetic equivalent,
characterized in that said graft is in the form of a coiled sheet, said coiled sheet (76) having the ability to uncoil in the subretinal space, said coiled sheet (76) having an axial length longer than its greatest cross-sectional diameter, and said coiled sheet being axially insertable through an incision in a host eye dimensioned to accommodate the cross-sectional area thereof, whereby said graft is positionable between the retina and the underlying tissue of the host eye, and then uncoiled to lie in sheet-like form between the retina and the underlying tissue of the host eye.

3. A graft according to claim 2, wherein said donor layer (54) is embedded in said carrier layer (70).

4. A graft as set forth in claim 2, wherein said graft is contained within an implement (78) for inserting said graft through an incision in a host eye, whereby the graft is positionable between the retina and the underlying tissue of the host eye.

## Patentansprüche

1. Verfahren zur Herstellung eines Implantats (74) mit einer schichtartigen Struktur zur Implantation in den Subretinalbereich eines Empfängerauges, wobei das Implantat eine Trägerschicht (70) aus einer nichttoxischen flexiblen Zusammensetzung aufweist, die sich im Empfängerauge im wesentlichen löst, und eine von einem Spenderauge erhaltene Donorschicht (54), wobei die Donorschicht (54) Augengewebe umfaßt, das ausgewählt ist aus Retinalgewebe, Epithelgewebe, Aderhautgewebe, und Bruch-Membran, oder einem synthetischen Äquivalent, und die Donorschicht (54) die gleiche zelluläre Struktur wie im Spenderauge aufweist, gekennzeichnet durch die Stufen:
- Bereitstellen eines rohrförmigen Körpers (90) mit einem ersten offenen Ende (92) und einem zweiten offenen Ende (96), wobei der Durchmesser des ersten offenen Endes größer ist als der Durchmesser des zweiten offenen Endes, und wobei die Bohrung des rohrförmigen Körpers einen trichterförmigen Abschnitt (94) aufweist, der vom ersten offenen Ende zum zweiten offenen Ende führt;
- Bereitstellen des Implantats (74) mit der schichtartigen Struktur; und
- Aufwickeln des schichtartigen Implantats (74), um ein spiralförmiges Implantat (76) auszubilden, indem man das schichtartige Implantat mit einem Ende zuerst in das erste offene Ende (92) des rohrförmigen Körpers gibt, und das schichtartige Implantat durch den rohrförmigen Körper (90) gegen das zweite offene Ende (96) einführt, wodurch das Zusammenwirken des schichtartigen Implantats (74) mit der Oberfläche der Bohrung, wenn dieses durch den trichterförmigen Abschnitt (94) hindurchläuft, das schichtartige Implantat in ein spiralförmiges Implantat (76) rollt, wobei das spiralförmige Implantat (76) aus dem zweiten Ende (96) austreten kann.

2. Transplantat zur Transplantation in den Subretinalbereich eines Empfängerauges, wobei das Transplantat eine Trägerschicht (70) aus einer nicht-toxischen flexiblen Zusammensetzung aufweist, die sich im Empfängerauge im wesentlichen löst, und eine von einem Spenderauge erhaltene Donorschicht (54), wobei die Donorschicht (54) die gleiche zelluläre Struktur wie im Spenderauge aufweist, und die Donorschicht (54) Augengewebe umfaßt, das ausgewählt ist aus Retinalgewebe, Epithelgewebe, Aderhautgewebe und Bruch-Membran, oder einem synthetischen Äquivalent,
dadurch gekennzeichnet, daß das Transplantat in Form einer spiralförmigen Folie vorliegt, wobei die spiralförmige Folie (76) die Fähigkeit besitzt, sich im Subretinalraum zu entrollen, und die spiralförmige Folie (76) eine achsiale Länge aufweist, die länger ist als der größte Querschnittsdurchmesser, und die spiralförmige Folie achsial durch einen Einschnitt in einem Empfängerauge einführbar ist, der so dimensioniert ist, daß er sich dem Querschnitt der spiralförmigen Folie anpaßt, wodurch das Transplantat zwischen der Retina und dem darunterliegenden Gewebe des Empfängerauges positionierbar ist, und sich dann entrollt, um schichtförmig zwischen der Retina und dem darunterliegenden Gewebe des Empfängerauges zu liegen.

3. Transplantat nach Anspruch 2, dadurch gekennzeichnet, daß die Donorschicht (54) in der Trägerschicht (70) eingebettet ist.

4. Transplantat nach Anspruch 2, dadurch gekennzeichnet, daß das Transplantat in einem Instrument (78) zum Einführen des Transplantats durch einen Einschnitt in ein Empfängerauge enthalten ist, wodurch das Transplantat zwischen der Retina und dem darunterliegenden Gewebe des Empfängerauges positionierbar ist.

## Revendications

1. Une méthode pour préparer un implant (74) ayant la configuration d'une feuille en vue de son implantation dans la région sub-rétinienne de l'oeil d'un hôte, dans laquelle ledit implant comprend une couche porteuse (70) en une composition flexible non toxique qui se dissout pratiquement dans l'oeil de l'hôte et une couche du donneur (54) recueillie sur l'oeil du donneur, ladite couche du donneur (54) comprenant du tissu de l'oeil choisi entre le tissu rétinien, le tissu épithélial, le tissu choroïdal et la membrane de Bruch ou un équivalent synthétique, ladite couche du donneur (54) ayant la même organisation cellulaire que dans l'oeil du donneur,
caractérisé en ce qu'elle comprend les étapes consistant à :
- prévoir un corps tubulaire (90) ayant une première extrémité ouverte (92) et une seconde extrémité ouverte (96), le diamètre de ladite première extrémité ouverte étant supérieur au diamètre de ladite seconde extrémité ouverte, et dans lequel la partie creuse dudit corps tubulaire comporte une portion en forme de cheminée (94) conduisant depuis ladite première extrémité ouverte vers ladite seconde extrémité ouverte ;
- prévoir ledit implant (74) ayant une configuration en forme de feuille ; et
- enrouler ledit implant en forme de feuille (74) de manière à former un implant en forme de volute (76) en plaçant d'abord une extrémité de l'implant en forme de feuille dans la première extrémité ouverte (92) dudit corps tubulaire, faire passer l'implant en forme de feuille à travers ledit corps tubulaire (90) vers ladite seconde extrémité ouverte (96), grâce à quoi la coopération dudit implant en forme de feuille (74) avec la surface de ladite partie creuse à mesure qu'il chemine à travers ladite portion en forme de cheminée (94) provoque l'enroulement de l'implant en forme de feuille en un implant en forme de volute (76), ledit implant en forme de volute (76) pouvant alors sortir par ladite seconde extrémité (96).

2. Une greffe destinée à la transplantation dans la région sub-rétinienne de l'oeil d'un hôte, dans laquelle ladite greffe comprend une couche porteuse (70) en une composition flexible non toxique qui se dissout pratiquement dans l'oeil de l'hôte, et une couche du donneur (54) recueillie dans l'oeil d'un donneur, ladite couche du donneur (54) ayant la même organisation cellulaire que dans l'oeil du donneur, ladite couche du donneur (54) comprenant du tissu de l'oeil choisi entre le tissu rétinien, le tissu épithélial, le tissu choroïdal et la membrane de Bruch, ou un équivalent synthétique,
caractérisé en ce que ladite greffe a la forme d'une feuille enroulée, ladite feuille enroulée (75) ayant l'aptitude à se dérouler dans l'espace sub-rétinien, ladite feuille enroulée (76) ayant axialement une longueur supérieure à son plus grand diamètre transversal, et ladite feuille enroulée pouvant être insérée axialement par une incision dans l'oeil d'un hôte dimensionnée de manière à pouvoir recevoir la région de la section transversale de celle-ci, grâce à quoi ladite greffe peut être amenée en position entre la rétine et le tissu sous-jacent de l'oeil de l'hôte, puis se dérouler pour reposer sous forme de feuille entre la rétine et le tissu sous-jacent de l'oeil de l'hôte.

3. Une greffe selon la revendication 2, dans laquelle ladite couche du donneur (54) est noyée dans ladite couche porteuse (70).

4. Une greffe selon la revendication 2, dans laquelle ladite greffe est contenue à l'intérieur d'un ustensile (78) destiné à insérer ladite greffe à travers une incision dans l'oeil d'un hôte, grâce à quoi la greffe peut être amenée en position entre la rétine et le tissu sous-jacent de l'oeil de l'hôte.
